# EUROPÄISCHE PATENTSCHRIFT

(11) **EP 2 412 698 B1**
(45) Veröffentlichungstag und Bekanntmachung des Hinweises auf die Patenterteilung: **21.11.2012**
(21) Anmeldenummer: 11174776.2
(22) Anmeldetag: 21.07.2011
(51) Int. Cl.: C07C 209/48, C07C 209/84, C07C 211/11, C07C 255/24, C07C 253/30

(54) **DMAPN mit niedrigem DGN-Gehalt und ein Verfahren zur Herstellung von DMAPA aus DMAPN mit niedrigem DGN-gehalt**
DMAPN with low DGN content and method for producing DMAPA from DMAPN with low DGN content
DMAPN ayant une teneur réduite en DGN et procédé de fabrication de DMAPA à partir de DMAPN ayant une teneur réduite en DGN

(30) Priorität: 29.07.2010 EP 10171187
(43) Veröffentlichungstag der Anmeldung: 01.02.2012
(73) Patentinhaber: BASF SE, 67056 Ludwigshafen (DE)
(72) Erfinder: Mägerlein, Wolfgang, 68165 Mannheim (DE); Eberhardt, Jan, 68167 Mannheim (DE); Melder, Johann-Peter, 67459 Böhl-Iggelheim (DE); Köhler, Ulrich, 68259 Mannheim (DE); Hahn, Thilo, 67292 Kirchheimbolanden (DE); Kreitschmann, Mirko, 68161 Mannheim (DE); Herbrecht, Dominik, 69124 Heidelberg (DE)

(56) Entgegenhaltungen:
- WO-A1-2007/128803

## Beschreibung

Die vorliegende Anmeldung schließt durch Verweis die am 29.07.2010 eingereichte vorläufige US-Anmeldung 61/368656 ein.

Die vorliegende Erfindung betrifft ein Verfahren zur Herstellung von 3-Dimethylaminopropropylamin (DMAPA). Des Weiteren betrifft die vorliegende Erfindung Mischungen von DMAPN und 2-(Dimethylaminomethyl)-glutaronitril (DGN) mit einem niedrigen DGN-Gehalt.

3-Dimethylaminopropylamin (DMAPA, N,N-Dimethyl-1,3-diaminopropan) stellt ein wichtiges Zwischenprodukt für die technische Produktion beispielsweise von Flüssigseifen dar. DMAPA dient zudem als Ausgangsprodukt für die Herstellung von Koagulationsmitteln und soll selbst antikorrosive Eigenschaften aufweisen.

DMAPA wird in der Regel durch einen zweistufigen Prozess hergestellt.

In der ersten Stufe wird üblicherweise Acrylnitril (ACN) mit Dimethylamin (DMA) umgesetzt, wobei sich in der Regel 3-Dimethylaminopropionitril (DMAPN) bildet.

DMAPN wird dann im Allgemeinen in einer weiteren Stufe zu DMAPA reduziert.

Gemäß der WO 2007/128803 ist es von Vorteil, wenn zur Herstellung von DMAPA ein integrales Herstellverfahren bzw. eine integrale Vorrichtung zum Einsatz kommt. Hierbei wird der zunächst erhaltene Produktstrom, welcher DMAPN aufweist, direkt oder nach Aufreinigung eingesetzt, um in einem weiteren Schritt zu DMAPA umgesetzt zu werden. Gemäß der Lehre der WO 2007/128803 ist hierbei für die Reduktionsreaktion von DMAPN zu DMAPA die Qualität des DMAPN-Produktstromes aus der ersten Umsetzung (DMA und ACN) von entscheidender Bedeutung, insbesondere für den Verbrauch des bei der Reduktion eingesetzten Katalysators. Die WO 2007/128803 lehrt deshalb ein Verfahren zur Herstellung von DMAPN durch Umsetzung von DMA mit ACN in kontinuierlicher Fahrweise, wobei zunächst DMA und anschließend ACN kontinuierlich zugeführt werden und die Umsetzung des Reaktionsstroms in einem ersten Reaktionsbereich und zumindest teilweise in einem zweiten Reaktionsbereich erfolgt.

Das so erhaltene DMAPN wird dann in der Regel ohne weitere Aufarbeitung in einem weiteren Reaktionsbereich mit Wasserstoff direkt zu DMAPA reduziert.

Offenbarungsgemäß kann bei der Hydrierung von DMAPN die Standzeit des Hydrierkatalysators verlängert werden, wenn man in die DMAPN-Hydrierung ein DMAPN einsetzt, dass gemäß der Lehre von WO 2007/128803 in zwei separaten Reaktionsräumen hergestellt wird.

Die Aufgabe der vorliegenden Erfindung gegenüber dem Stand der Technik bestand darin, die Standzeit der in die Hydrierung von DMAPN eingesetzten Katalysatoren weiter zu verbessern. Insbesondere sollte erreicht werden, dass unerwünschte Nebenreaktionen, wie die exotherme Zersetzung des Nitrils, die Rückspaltung von DMAPN zu ACN und DMA oder die Bildung sekundärer Amine durch Kondensation von DMAPA während des Einsatzes des Katalysators gering gehalten werden können, ohne die Katalysatorbelastung erniedrigen zu müssen. Aus technischer Sicht ist ein geringer Gehalt von DMAPN im Produkt vorteilhaft, da DMAPN von DMAPA nur schwer abzutrennen ist und in den Folgeanwendungen zu unerwünschten Eigenschaften, wie Geruch und Verfärbung, führt. Der Gehalt von DMAPN im Hydrieraustrag kann zwar zu einem gewissen Grad durch die Erhöhung der Reaktionstemperatur gegenüber dem Anfangswert kompensiert werden, da dies zu einem höheren Umsatz führt, aber durch die Erhöhung der Reaktionstemperatur entstehen andere Nebenkomponenten, die qualitätsmindernd wirken. Aus diesem Grund kann die Reaktionstemperatur deshalb nicht beliebig erhöht werden. Dementsprechend bestand eine Aufgabe der vorliegenden Erfindung darin, die Katalysatordeaktivierung während des Prozesses gering zu halten, um somit die Lebensdauer des Katalysators bei gleichbleibend guter Leistungsfähigkeit des Katalysators zu verlängern. Ein Maß für diese Eigenschaften ist der Quotient aus Temperaturerhöhung und Betriebsdauer. Ein geringer Quotient aus Temperaturerhöhung und Betriebsdauer bedeutet, dass der Katalysator möglichst lange bei der Ausgangstemperatur betrieben werden kann und dass die erforderliche Temperaturerhöhung, die ggf. zur Kompensation der Katalystordeaktivierung erforderlich ist, gering ausfällt Somit werden kritische Temperaturen, bei denen die Nebenreaktionen zu sehr zunehmen, möglichst lange nicht erreicht. Deshalb war es auch ein Ziel der vorliegenden Erfindung den Quotienten aus Temperaturerhöhung und Betriebsdauer des Katalysators zu minimieren.

Die Aufgabe der vorliegenden Erfindung konnte gelöst werden, durch ein Verfahren zur Herstellung von 3-Dimethylaminopropylamin (DMAPA) durch Umsetzung von 3-Dimethylaminoprionitril (DMAPN) mit Wasserstoff in Gegenwart eines Katalysators, dadurch gekennzeichnet, dass das eingesetzte DMAPN einen Gehalt an 2-(Dimethylaminomethyl)-glutaronitril (DGN) von 300 Gew.-ppm oder weniger enthält, bezogen auf das eingesetzte DMAPN.

Überraschenderweise wurde gefunden, dass 2-(Dimethylaminomethyl)-glutaronitril (DGN) der Formel (I) 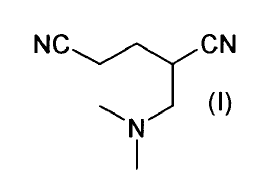 im DMAPN zu einer schnelleren Deaktivierung von Nitril-Hydrierkatalysatoren führt. Wenn der Gehalt an DGN einen Wert von 300 ppm dagegen nicht überschreitet, kann gegenüber dem Stand der Technik eine weitere Erhöhung der Standzeit erreicht werden. Zwar offenbart WO 2007/128803, dass die Qualität des in die Hydrierung eingesetzten DMAPN maßgeblich für die Qualität des durch Hydrierung von DMAPN erhaltenen DMAPAs sein kann, es erfolgt aber kein Hinweis darauf, welche Kenngrößen des DMAPN die Hydrierreaktion beeinflussen. Weiterhin wird mittels dem in WO 2007/128803 offenbarten Verfahren nicht direkt und unmittelbar ein DMAPN mit einem DGN-Gehalt von 300 ppm und weniger offenbart. Auch ist in anderen Dokumenten des Stands der Technik (z.B. der WO-A-2007/051786, DD-A-58306, DD-A-222011, US 4,172,091) weder ein eindeutiger und unmittelbarer Hinweis enthalten, wie man ein DMAPN mit einem DGN-Gehalt von 300 ppm herstellen kann, noch dass sich der Gehalt an DGN im DMAPN auf die nachfolgende Hydrierung auswirkt.

In das erfindungsgemäße Verfahren wird ein DMAPN mit einem Gehalt von 300 ppm oder weniger DGN, bezogen auf das eingesetzte DMAPN, eingesetzt. Bevorzugt wird in das erfindungsgemäße Verfahren ein DMAPN mit einem Gehalt von DGN von 250 ppm und weniger, besonders bevorzugt 150 ppm und weniger und insbesondere bevorzugt 50 ppm und weniger eingesetzt.

In einer bevorzugten Ausführungsform kann DMAPN mit einem Gehalt von 300 ppm DGN oder weniger durch kontinuierliche Umsetzung von ACN mit DMA und anschließender Destillation hergestellt werden. In dieser bevorzugten Ausführungsform kann die Herstellung von DMAPN prinzipiell analog der Lehre der WO 2007/128803 oder der Lehre der WO 2007/051786 erfolgen, auf deren Inhalt ausdrücklich Bezug genommen wird.

Bevorzugt erfolgt die Umsetzung von ACN mit DMA in dieser bevorzugten Ausführungsform in einer Reaktorkaskade, wobei bevorzugt 2 bis 10, besonders bevorzugt 2 bis 8 und ganz besonders bevorzugt 3 bis 7 Reaktoren hintereinandergeschaltet werden. Die Umsetzung von DMA mit ACN in einer Reaktorkaskade ermöglicht die Einstellung von unterschiedlichen Reaktionsbedingungen. Hierdurch kann eine vollständigere Umsetzung von ACN bei gleichzeitiger Verminderung des Anteils an Nebenprodukten im Reaktionsstrom erreicht werden.

Die Reaktoren der Reaktorkaskade können beispielsweise Rührkesselreaktoren, Reaktoren mit externem Umpumpkreislauf (Schlaufenreaktoren) oder Rohreaktoren sein, wobei es bevorzugt ist, dass der erste oder die ersten Reaktoren der Reaktorkaskade Rührkesselreaktoren sind und der letzte oder die letzten Reaktoren der Reaktorkaskade Rohreaktoren sind. Vorzugsweise liegt die Reaktionstemperatur in der bevorzugten Ausführungsform in den Reaktoren im Bereich von 20 °C bis 120 °C. Mehr bevorzugt beträgt der Bereich 40 °C bis 90 °C, wobei es weiterhin bevorzugt ist, dass die Temperatur im letzten Reaktor bzw. in den letzten Reaktoren der Reaktorkaskade eine niedrigere Reaktionstemperatur aufweist als in dem ersten bzw. den ersten Reaktoren der Reaktorkaskade.

In der bevorzugten Ausführungsform erfolgt die Umsetzung von ACN und DMA bevorzugt in einem Druckbereich von 1 bar bis 20 bar, bevorzugt 2 bis 15 bar und besonders bevorzugt 3 bis 8 bar, wobei der Druck in der Regel durch den Eigendruck des eingesetzten DMAs bei den jeweiligen Reaktionsbedingungen bestimmt wird.

In einer ganz besonders bevorzugten Ausführungsform erfolgt die Umsetzung von ACN mit DMA in Gegenwart von Wasser.

Wenn die Umsetzung in Gegenwart von Wasser erfolgt, dann ist es bevorzugt, dass der Anteil von Wasser, bezogen auf die Summe des eingesetzten ACNs und DMAs, im Bereich von 0,5 bis 10 Gew.-%, besonders bevorzugt im Bereich von 1 bis 8 Gew.-% und ganz besonders bevorzugt im Bereich von 3 bis 7 Gew.-% liegt. Das molare Verhältnis von DMA zu ACN beträgt dann bevorzugt 1,00: 1 bis 1,10:1, bevorzugt 1,01:1 bis 1,08:1 und besonders bevorzugt 1,02:1 1 bis 1,06:1.

Wird in die Umsetzung kein zusätzliches Wasser eingesetzt, ist es vorteilhaft einen molaren DMA-Überschuss einzusetzen. Das molare Verhältnis von DMA zu ACN beträgt dann bevorzugt mehr als 1,02: 1, besonders bevorzugt 1,04: 1 bis 1,35:1 und ganz bevorzugt 1,06:1 bis 1,25:1.

In der bevorzugten Ausführungsform kann die Zuführung von ACN und DMA und optional Wasser zusammen oder getrennt voneinander erfolgen. Die Zuführung von DMA und ACN erfolgt bevorzugt getrennt voneinander. Besonders bevorzugt erfolgt die Zuführung in der Art, dass zunächst DMA in einen Umlaufstrom eingeleitet wird, der bereits DMAPN enthält und die Zuführung von ACN bevorzugt im Anschluss an die Zusammenführung von DMA und DMAPN erfolgt. Das Wasser kann beispielsweise in Form einer zusätzlichen Zuführung in den Reaktionsstrom gelangen. Weiterhin ist es möglich, dass zumindest teilweise DMA in Form einer wässrigen Lösung dem Reaktionsstrom zugeführt wird. Dies kann beispielsweise in Form einer 30 bis 70 gew.-%igen wässrigen DMA-Lösung erfolgen. Zudem kann das eingesetzte ACN Wasser enthalten.

Es hat sich weiterhin als vorteilhaft erwiesen, wenn DMA und ACN in flüssiger Form zugeführt werden. Dies erlaubt einen höheren Umsatz bei besserer Raum/Zeit-Ausbeute im Vergleich zu Verfahren, bei denen DMA in gasförmigem Zustand dem Reaktionsgemisch zugeführt wird. Daher weist die Zuführung von ACN sowie von DMA eine geeignete Temperatur sowie einen geeigneten Druck auf, die gewährleisten, dass diese Edukte zur Herstellung von DMAPN in flüssiger Form vorliegen.

In der bevorzugten Ausführungsform wird durch die kontinuierliche Umsetzung von ACN mit DMA ein DMAPN-haltiges Reaktionsgemisch als Reaktionsaustrag erhalten, der neben DMAPN in der Regel noch nicht umgesetztes DMA, DGN und andere organische Verbindungen, sowie ggf. Wasser und ggf. nicht umgesetztes ACN enthalten kann.

Ein Teil des DMAPNs kann als Kreislaufstrom oder Umlaufstrom in das Verfahren zurückgeführt werden, wobei die Rückführung bevorzugt nach dem ersten Reaktor erfolgt.

Das in dieser bevorzugten Ausführungsform erhaltene DMAPN-Reaktionsgemisch wird anschließend in eine thermische Trennsequenz eingeleitet, in welcher leichtflüchtige als auch schwersiedende Nebenkomponenten von DMAPN abgetrennt werden.

Hierfür kann bevorzugt eine Kolonnenverschaltung bestehend aus einer Kolonne für die Abtrennung von Leichtsiedern und einer weiteren Destillationskolonne zur Abtrennung der schwerer flüchtigen Nebenkomponenten verwendet werden.

Besonders bevorzugt kann die Abtrennung der Leicht- und Schwersieder in einer Kolonne durchgeführt werden, wobei Leichtsieder über Kopf, Schwersieder über Sumpf und DMAPN über einen gas- oder dampfförmigen Seitenabzug gewonnen werden.

In einer weiteren besonders bevorzugten Ausführungsform kann die Abtrennung der Leicht- und Schwersieder in einer Kolonne mit Trennwand im mittleren Teil erfolgen, bei der Leichtsieder über Kopf, Schwersieder über Sumpf und DMAPN auf der dem Zulauf gegenüberliegenden Seite der Trennwand gewonnen werden.

Leichtsieder (LS) wie z.B. DMA, ACN, ggf. Wasser und andere leichtsiedende Nebenkomponenten werden am Kolonnenkopf der Leichtsiederkolonne bzw. des Verstärkungsteils gewonnen. Schwersieder (SS) bezüglich DMAPN sind DGN und ggf. andere Nebenkomponenten mit niedrigerem Dampfdruck. Diese werden über den Sumpfaustrag des Abtriebsteiles bzw. der Abtriebskolonne abgetrennt.

Die genauen Betriebsbedingungen der Destillationssequenz können entsprechend der Trennleistung der verwendeten Kolonneneinbauten vom Fachmann anhand von gemessenen oder geschätzten Dampfdrücken und Dampf-Flüssig-Phasengleichgewichten (VLE) der in die Destillationssequenz eingeleiteten Komponenten nach herkömmlichen Berechnungsmethoden ermittelt werden.

Bevorzugt weist die Destillationssequenz Einbauten zur Erhöhung der Trennleistung auf. Die destillativen Einbauten können beispielsweise als Böden, Füllkörperschüttungen oder geordnete Packung vorliegen. Bevorzugt für eine Trennwandkolonne werden geordnete Packungen verwendet. Vorteilhaft bei der Verwendung geordneter Packungen ist der geringe Druckverlust und der geringe spezifische Flüssig-Hold-up im Vergleich zu Böden wie z.B. Ventilböden. Die Einbauten können in ein oder mehreren Betten vorliegen.

Der Reaktionsaustrag aus der DMAPN-Herstellung wird vorzugsweise in einem räumlichen Bereich zwischen 25% und 75% der theoretischen Böden der Destillationskolonne zugeführt (von unten gezählt). Beispielsweise kann die Zuführung etwas unterhalb der Mitte der theoretischen Böden erfolgen. Die optimale Zulaufstelle kann vom Fachmann mit den üblichen Berechnungswerkzeugen ermittelt werden.

Die Anzahl der theoretischen Trennstufen zur Abtrennung der Leichtsieder, der sogenannte Verstärkungsteil, liegt im Allgemeinen im Bereich von 10 bis 50, vorzugsweise 10 bis 30. Die Anzahl der theoretischen Trennstufen zur Abtrennung der Schwersieder, der sogenannte Abtriebsteil, liegt im Allgemeinen im Bereich von 10 bis 50, vorzugsweise 10 bis 30.

Der Kopfdruck der Destillationskolonne(n) beträgt besonders bevorzugt 0,1 bis 10 bar, besonders bevorzugt 0,5 bis 5 bar. Ganz besonders bevorzugt wird die Destillationskolonne(n) bei Normaldruck betrieben.

Erfolgt die Abtrennung der Leicht- und Schwersieder in einer Kolonnenverschaltung, bestehend aus einer Kolonne für die Abtrennung von Leichtsiedern und einer weiteren Destillationskolonne zur Abtrennung der schwerer flüchtigen Nebenkomponenten, so wird bevorzugt eine Temperatur im Sumpf der Leichtsiederkolonne eingestellt, die oberhalb der Verdampfungstemperatur von Wasser aber unterhalb der Verdampfungstemperatur von DMAPN liegt. Beispielsweise kann bei einem Kopfdruck von 1 bar (abs) bevorzugt eine Kolonnensumpftemperatur von 100 bis 170°C, besonders bevorzugt 110 bis 150°C eingestellt werden. Im Sumpf der Schwersiederkolonne wird bevorzugt eine Temperatur eingestellt, die oberhalb der Verdampfungstemperatur von DMAPN aber unterhalb der Verdampfungstemperatur von DGN liegt. Beispielsweise kann in der Abtriebskolonne bei einem Kolonnenkopfdruck von 1 bar (abs.), bevorzugt eine Kolonnensumpftemperatur von 170 bis 200°C, besonders bevorzugt von 180 bis 195°C eingestellt werden.

Der Kondensator der Leichtsiederkolonne wird in der Regel bei einer Temperatur betrieben, in dem der überwiegende Teil des DMAs bzw. Wassers bei dem entsprechenden Kopfdruck kondensiert wird. In der Regel liegt die Betriebstemperatur des Kondensators im Bereich von 25 bis 100°C, vorzugsweise 25 bis 50°C.

Vorzugsweise wird das am Kondensator anfallende Kondensat zu mehr als 30 %, bevorzugt zu mehr als 50 Gew.-%, in den Kopf der Leichtsiederkolonne zurückgeführt. Die für die Verdampfung erforderliche Energie wird üblicherweise durch einen innen oder außen liegenden Verdampfer im Kolonnensumpf eingetragen.

Im Kondensator der Leichtsiederkolonne fällt ein Kondensat an, welches überwiegend DMA und ggf. Wasser und ggf. andere leichtsiedende organische Nebenkomponenten enthält. Dieses Kondensat kann als Ausgangsprodukt in die Reaktion zur Synthese von DMAPN zurückgeführt werden.

Im Sumpfaustrag der Leichtsiederkolonne wird DMAPN als Sumpfprodukt mit DGN und ggf. anderen schwer flüchtigen Nebenkomponenten erhalten.

Im Sumpfaustrag der Abtriebskolonne wird DGN als Sumpfprodukt und ggf anderen schwer flüchtigen Nebenkomponenten erhalten (DGN-Abtrennung).

DMAPN wird am Kopf der Abtriebskolonne gewonnen.

Erfolgt die Abtrennung der Leicht- und Schwersieder in einer Destillationskolonne, in der Leichtsieder über Kopf, Schwersieder über Sumpf und DMAPN über einen flüssigen oder dampfförmigen Seitenabzug gewonnen werden oder in einer Kolonne mit Trennwand im mittleren Teil, bei der Leichtsieder über Kopf, Schwersieder über Sumpf und DMAPN auf der dem Zulauf gegenüberliegenden Seite der Trennwand gewonnen werden, so wird bevorzugt eine Temperatur im Sumpf der Kolonne eingestellt, die oberhalb der Verdampfungstemperatur von DMAPN aber unterhalb der Verdampfungstemperatur von DGN liegt. Beispielsweise kann bei einem Kolonnenkopfdruck von 1 bar (abs.), bevorzugt eine Kolonnensumpftemperatur von 170 bis 200°C, besonders bevorzugt von 180 bis 195°C eingestellt werden.

Der Kondensator der Destillationskolonne wird in der Regel bei einer Temperatur betrieben, in dem der überwiegende Teil des DMAs bzw. Wassers bei dem entsprechenden Kopfdruck kondensiert wird.

In der Regel liegt die Betriebstemperatur des Kondensators im Bereich von 25 bis 100°C, vorzugsweise 25 bis 50°C.

Vorzugsweise wird das am Kondensator anfallende Kondensat zu mehr als 30 %, bevorzugt zu mehr als 50 Gew.-%, in den Kopf der Destillationskolonne zurückgeführt. Die für die Verdampfung erforderliche Energie wird üblicherweise durch einen innen oder außen liegenden Verdampfer im Kolonnensumpf eingetragen.

Im Kondensator fällt ein Kondensat an, welches überwiegend DMA und ggf. Wasser und ggf. andere leichtsiedende organische Nebenkomponenten enthält.

Dieses Kondensat kann als Ausgangsprodukt in die Reaktion zur Synthese von DMAPN zurückgeführt werden.

Im Sumpfaustrag des Abtriebsteiles wird DGN als Sumpfprodukt und ggf anderen schwer flüchtigen Nebenkomponenten erhalten (DGN-Abtrennung).

DMAPN wird über einen flüssigen oder dampfförmigen Seitenabzug gewonnen bzw. auf der dem Zulauf gegenüberliegenden Seite der Trennwand.

Das DMAPN, das als Produkt der Trennsequenz anfällt, hat üblicherweise einen Gehalt von mehr als 95 Gew.-% DMAPN, bevorzugt mehr als 98 Gew.-% DMAPN und besonders bevorzugt mehr als 99 Gew.-% DMAPN.

Das in dieser bevorzugten Ausführungsform erhaltene DMAPN weist bevorzugt einen DGN-Gehalt von 300 ppm oder weniger bezogen auf DMAPN auf, besonders bevorzugt einen Gehalt von 250 ppm oder weniger bezogen auf DMAPN, ganz besonders bevorzugt einen Gehalt von 150 ppm oder weniger bezogen auf DMAPN und insbesondere bevorzugt einen Gehalt von 50 ppm oder weniger bezogen auf DMAPN.

In einer ganz besonders bevorzugten Ausführungsform erfolgt die Umsetzung von Dimethylamin und Acrylnitril in Gegenwart von Wasser in einer Reaktorkaskade in einem Temperaturbereich von 50 bis 80°C und einem Druckbereich von 3 bis 8 bar, wobei das molare Verhältnis von DMA zu ACN im Bereich von 1,02:1 bis 1,10:1 liegt und der Anteil an Wasser im Bereich von 1 bis 8 Gew.-%, bezogen auf die Summe des eingesetzten ACN und DMA, liegt. Werden diese Reaktionsbedingungen eingehalten, so ist in der Regel eine destillative Aufarbeitung des Reaktionsaustrags aus der DMAPN-Herstellung nicht erforderlich, um ein DMAPN mit einen Gehalt an DGN von 300 ppm oder weniger zu erhalten.

Das Gewichtsverhältnis von DMAPN zu DGN im so erhaltenen DMAPN liegt bevorzugt im Bereich von 1 000 000 : 1 bis 1 000 000 : 300, besonders bevorzugt im Bereich von 1 000 000 : 5 bis 1 000 000 : 250, ganz besonders bevorzugt im Bereich von 1 000 000 : 8 bis 1 000 000 : 150 und insbesondere bevorzugt im Bereich von 1 000 000 : 10 bis 1 000 000 : 100 . Demgemäß betrifft die vorliegende Erfindung auch eine Mischung von DMAPN und DGN, dadurch gekennzeichnet, dass das Gewichtsverhältnis von DMAPN zu DGN im Bereich von 1 000 000 : 5 bis 1 000 000 : 250 liegt.

Der Gehalt von DGN in DMAPN kann leicht durch die dem Fachmann bekannten Analyseverfahren ermittelt werden, beispielsweise durch Gaschromatographie.

In das erfindungsgemäße Verfahren zur Herstellung von DMAPA wird weiterhin Wasserstoff eingesetzt.

Als Reduktionsmittel können Wasserstoff oder ein Wasserstoff enthaltendes Gas verwendet werden. Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

Die Umsetzung von DMAPN mit einem DGN-Gehalt von 300 ppm oder weniger und Wasserstoff kann in Gegenwart von Ammoniak erfolgen. Bevorzugt wird in das Verfahren reiner Ammoniak eingesetzt, bevorzugt Ammoniak mit einem Gehalt von mehr als 99 Gew.-% Ammoniak und besonders bevorzugt mehr als 99,9 Gew.-% Ammoniak.

In einer bevorzugten Ausführungsform wird Ammoniak in der Regel dabei in Molverhältnissen zur Nitrilgruppe im Verhältnis von 0,5 : 1 bis 100 : 1, vorzugsweise 2 : 1 bis 20 : 1 eingesetzt. Eine weitere bevorzugte Ausführungsform ist ein Verfahren, bei dem kein Ammoniak zugegeben wird.

Die Umsetzung von DMAPN mit einem DGN-Gehalt von 300 ppm oder weniger mit Wasserstoff erfolgt in Gegenwart eines Katalysators.

Als Katalysatoren zur Hydrierung der Nitril-Funktion zum Amin können insbesondere Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind sogenannte Skelett-Katalysatoren (auch als Raney^{®}-Typ bezeichnet, nachfolgend auch: Raney-Katalysator), die durch Auslaugen (Aktivierung) einer Legierung aus hydrieraktivem Metall und einer weiteren Komponente (bevorzugt AI) erhalten werden. Die Katalysatoren können zusätzlich einen oder mehrere Promotoren enthalten. In einer besonders bevorzugten Ausführungsform werden Raney-Nickel-Katalysatoren oder Raney-Cobalt-Katalysatoren eingesetzt.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruß, Graphit) zur Anwendung.

Die oxidischen Katalysatoren werden vor dem Einsatz außerhalb des Reaktors oder im Reaktor durch Reduktion der Metalloxide in einem Wasserstoff enthaltenden Gasstrom bei erhöhter Temperatur aktiviert. Wenn die Katalysatoren außerhalb des Reaktors reduziert werden, kann danach eine Passivierung durch einen Sauerstoff enthaltenden Gasstrom oder die Einbettung in ein inertes Material erfolgen, um eine unkontrollierte Oxidation an Luft zu vermeiden und einen sicheren Umgang zu ermöglichen. Vor dem Einsatz der passivierten Katalysatoren in die Reaktion werden diese üblicherweise mit wasserstoffenthaltendem Gas bei erhöhter Temperatur reaktiviert.

Besonders bevorzugte Festbett-Katalysatoren sind die in EP-A 0 742 045 bzw. EP-A-0 636 409 offenbarten Cobalt-Vollkontakte, dotiert mit Mn, P und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A-1306365 offenbarten Katalysatoren, enthaltend Cobalt und gegebenenfalls zusätzlich Nickel sowie mindestens ein weiteres Dotiermetall auf einem teilchenförmigen Trägermaterial, dadurch gekennzeichnet, dass das Cobalt und gegebenenfalls das Nickel eine mittlere Teilchengröße von 3 bis 30 nm im aktiven Katalysator aufweisen.

Das erfindungsgemäße Verfahren zur Herstellung von DMAPA erfolgt bevorzugt in kontinuierlicher Fahrweise. Dies ermöglicht einen besonders effizienten Umsatz verbunden mit einer hohen Raum/Zeit-Ausbeute. Die Herstellung von DMAPA kann aber auch diskontinuierlich oder semikontinuierlich erfolgen.

Typische Reaktoren, in denen die Hydrierung durchgeführt werden kann, sind beispielsweise Hochdruck-Rührkessel-Reaktoren, Schlaufenreaktoren, Autoklaven oder Festbettreaktoren. Bevorzugt wird das erfindungsgemäße Verfahren in einem Hochdruck-Rührkessel-Reaktor oder Festbettreaktor durchgeführt. Ganz besonders bevorzugt wird die Hydrierung in einem Festbettreaktor durchgeführt.

Der Festbettreaktor kann von oben nach unten (Rieselfahrweise) oder unten nach oben (Sumpffahrweise) durchströmt werden.

Wasserstoff, DMAPN und ggf. Ammoniak können gemeinsam in die Reaktionszone des Reaktors gegeben werden, zum Beispiel als vorgemischter Reaktandenstrom, oder getrennt.
Es kann weiterhin ein Rückführstrom bzw. Umlaufstrom zugefahren werden.

Die Hydrierung wird in der Regel bei einem Druck von 1 bis 500 bar, bevorzugt 10 bis 400 bar, besonders bevorzugt bei einem Druck von 20 bis 300 bar und ganz besonders bevorzugt 30 bis 200 bar durchgeführt. Die Druckhaltung bzw. Drucksteuerung erfolgt in der Regel über die Dosierung des Wasserstoffs.

Die Hydrierung erfolgt im Allgemeinen bei Temperaturen von 20 bis 400°C, bevorzugt 40 bis 250°C, besonders bevorzugt 50 bis 180°C und ganz besonders bevorzugt 70 bis 150°C.

Der Reaktionsaustrag aus dem Hydrierreaktor wird bevorzugt über einen Wärmetauscher geleitet. Nach Überleitung über den Wärmetauscher liegt die Temperatur des Hydrieraustrags bevorzugt im Bereich von 20 bis 80°C.

In einer bevorzugten Ausführungsform erfolgt die Hydrierung adiabatisch.

Der Hydrieraustrag wird üblicherweise im Anschluss an die Hydrierung aufgearbeitet. Im Allgemeinen werden Wasserstoff und Ammoniak vom Hydrieraustrag getrennt und können nach der Abtrennung wieder in den Prozess zurückgeführt werden.

Bevorzugt wird der Hydrieraustrag zur Aufarbeitung in einen Abscheider geleitet, in dem flüssiges Rohprodukt von gasförmigen Komponenten, wie Wasserstoff, DMA und Ammoniak, abgetrennt wird und über einen Auslass ausgeführt werden kann.

In einer bevorzugten Ausführungsform kann das Rohprodukt zumindest teilweise dem Reaktionsstrom in einer Kreisfahrweise wieder zugeführt werden.

Das anfallende Gemisch aus Wasserstoff, DMA und Ammoniak kann über einen weiteren Auslass aus dem Phasenscheider ausgeführt werden und ebenfalls bevorzugt in den Prozess oder ggf. nach Auftrennung in die DMAPN-Herstellung zurückgeführt werden.

Die vorliegende Erfindung ermöglicht gegenüber dem Stand der Technik die Standzeit der in die Hydrierung von DMAPN eingesetzten Katalysatoren weiter zu verbessern.

Insbesondere können unerwünschte Nebenreaktionen, wie die exotherme Zersetzung des Nitrils, die Rückspaltung von DMAPN zu ACN und DMA oder die Bildung von sekundären Aminen durch Kondensation von DMAPA während des Einsatzes des Katalysators gering gehalten werden, ohne dass die Katalysatorbelastung erniedrigt werden muss. Aus technischer Sicht ist ein geringer Gehalt von DMAPN im Produkt vorteilhaft, da DMAPN von DMAPA nur schwer abzutrennen ist und in den Folgeanwendungen zu unerwünschten Eigenschaften, wie Geruch und Verfärbung führt. Der Gehalt von DMAPN im Hydrieraustrag kann zwar zu einem gewissen Grad durch die Erhöhung der Reaktionstemperatur gegenüber dem Anfangswert kompensiert werden, da dies zu einem höheren Umsatz führt, aber durch die Erhöhung der Reaktionstemperatur entstehen andere Nebenkomponenten, die qualitätsmindernd wirken, so dass die Reaktionstemperatur nicht beliebig erhöht werden kann. Das erfindungsgemäße Verfahren ermöglicht es die Katalysatordeaktivierung während des Prozesses gering zu halten, um somit die Lebensdauer des Katalysators bei gleichbleibend guter Leistungsfähigkeit des Katalysators zu verlängern. Ein Maß für diese Eigenschaften ist der Quotient aus Temperaturerhöhung und Betriebsdauer. Ein geringer Quotient aus Temperaturerhöhung und Betriebsdauer bedeutet, dass der Katalysator möglichst lange bei der Ausgangstemperatur betrieben werden kann und dass die erforderliche Temperaturerhöhung, der ggf. zur Kompensation der Katalystordeaktivierung erforderlich ist, gering ausfällt, so dass eine kritische Temperatur, bei der die Nebenreaktionen zu sehr zunehmen, möglichst lange nicht erreicht wird. Durch das erfindungsgemäße Verfahren kann der Quotient aus Temperaturerhöhung und Betriebsdauer des Katalysators verringert werden.

Die vorliegende Erfindung wird anhand der folgenden Beispiele erläutert.

### Beispiele

Beispiele a) bis c): Herstellung von 3-Dimethylaminopropionitril (DMAPN) als Einsatzware für die Beispiele 1 - 3:
Allgemeine Durchführung:
   Die Herstellung von DMAPN erfolgte durch Reaktion von Acrylnitril (ACN) und Dimethylamin (DMA), optional in Gegenwart von geringen Mengen Wasser.

Es wurde eine kontinuierliche Apparatur verwendet, die aus einer Kaskade von drei hintereinander geschalteten Rührautoklaven bestand (C1 bis C3). C1 besaß ein Volumen von 80 ml, C2 besaß ein Volumen von 120 ml und C3 besaß ein Volumen von 200 ml. Optional konnten nach C3 noch vier weitere Rührautoklaven in Reihe geschalten werden: C4, C5, C6 und C7 mit einem Volumen von jeweils 270 ml. Unter Volumen wird das mit Flüssigkeit gefüllte Volumen verstanden.

ACN, DMA und optional Wasser wurden gleichzeitig in die Anlage vor C1 dosiert. Die Fahrweise erfolgte im geraden Durchgang. Der Druck in den Autoklaven betrug 5 bar.

Das so hergestellte DMAPN wurde ohne weitere Aufarbeitung direkt in die Hydrierstufe (Beispiele 1 bis 3) eingespeist. Ein Teil des Roh-DMAPN wurde abgetrennt und bzgl. Nebenkomponenten analysiert.

Beispiel a): Herstellung von DMAPN für Beispiel 1:
Verwendung von C1, C2 und C3
Temperatur C1: 60 °C; Temperatur C2: 70 °C; Temperatur C3: 70 °C
Dosierung ACN: 43.3 g/h
Dosierung DMA: 37.5 g/h (Überschuß von 2 mol% DMA bzgl. ACN)
Dosierung Wasser: 2,1 g/h (entspricht 2,6 Gew.-% bezogen auf die eingesetzte Menge DMA und ACN)

Analytik bzgl. Nebenkomponenten:
Zur Analytik der schwersiedenden Nebenprodukte wurde eine Menge von 1403 g des so hergestellten DMAPN in einer Destillationsapparatur mit Vigreux-Kolonne bei 100 mbar und 100 - 105 °C Kopftemperatur und bis zu max. 135 °C Sumpftemperatur bis auf einen Rückstand von 10 - 15 g eingeengt.

Von diesem Rückstand wurde eine GC-Analyse durchgeführt:
GC-Säule: 30 m RTX-5; ID = 0,32 mm, Filmdicke = 1,5µm
Temperaturprogramm: 70 °C - 7 °C/min - 280 °C - 30 min
DGN wurde bei einer Retentionszeit von 16 min detektiert.
Der Gehalt an DGN, bezogen auf die ursprünglich in die Destillation eingesetzte Menge DMAPN (1403 g) betrug 227 ppm.

Beispiel b) Herstellung von DMAPN für Beispiel 2:
Verwendung von C1, C2, C3, C4, C5, C6 und C7
Temperatur C1: 60 °C; Temperatur C2: 70 °C; Temperatur C3: 70 °C; Temperatur C4: 70 °C;
Temperatur C5: 70 °C; Temperatur C6: 70 °C; Temperatur C7: 60 °C
Dosierung ACN: 43.3 g/h
Dosierung DMA: 37.5 g/h (Überschuß von 2 mol% DMA bzgl. ACN)

Analytik bzgl. Nebenkomponenten:
Zur Analytik der schwersiedenden Nebenprodukte wurde eine Menge von 1406 g des so hergestellten DMAPN in einer Destillationsapparatur mit Vigreux-Kolonne bei 100 mbar und 100 - 105 °C Kopftemperatur und bis zu max. 135 °C Sumpftemperatur bis auf einen Rückstand von 10 g eingeengt.

Von diesem Rückstand wurde eine GC-Analyse durchgeführt (vgl. Beispiel a)):
Der Gehalt an DGN, bezogen auf die ursprünglich in die Destillation eingesetzte Menge DMAPN (1406 g) betrug 373 ppm.

Beispiel c) Herstellung von DMAPN für Beispiel 3:
Verwendung von C1 , C2, C3, C4, C5, C6 und C7
Temperatur C1: 60 °C; Temperatur C2: 70 °C; Temperatur C3: 70 °C; Temperatur C4: 70 °C;
Temperatur C5: 70 °C; Temperatur C6: 70 °C; Temperatur C7: 60 °C
Dosierung ACN: 43.3 g/h
Dosierung DMA: 37.5 g/h (Überschuß von 2 mol% DMA bzgl. ACN)
Dosierung Wasser: 4 g/h

Zur Analytik der schwersiedenden Nebenprodukte wurde eine Menge von 1014 g des so hergestellten DMAPN in einer Destillationsapparatur mit Vigreux-Kolonne bei 100 mbar und 100 - 105 °C Kopftemperatur und bis zu max. 135 °C Sumpftemperatur bis auf einen Rückstand von 5,3 g eingeengt.

Von diesem Rückstand wurde eine GC-Analyse durchgeführt (vgl. Beispiel a)):
Der Gehalt an DGN, bezogen auf die ursprünglich in die Destillation eingesetzte Menge DMAPN (1014 g) betrug 27 ppm.

### Beispiele 1 bis 3): Hydrierung des DMAPN aus den Beispielen a) bis c)

Allgemeine Durchführung:
Die kontinuierliche Hydrierung von DMAPN zu DMAPA wurde in einem senkrecht aufgebauten Rohrreaktor mit einer beheizbaren Länge von 100 cm und einem Innendurchmesser von 6 mm durchgeführt (Reaktorvolumen ca. 28 mL).

Der Reaktor wurde mit 26 g eines Cobaltkatalysators befüllt, dessen Herstellung in der EP-A-0636409 beschrieben ist (Beispiel Katalysator A).

Durch Auflösen von Kobaltnitrat, Mangannitrat und Phosphorsäure in Wasser wurde eine Lösung hergestellt, die 10 Gew.-% Kobalt, 0,55 Gew.-% Mangan und 0,45 Gew.-% H₃PO₄ enthält. Durch Zugabe einer 20%igen Natriumcarbonatlösung wurde bei einer Temperatur von 50°C gefällt. Der entstandene Niederschlag wurde gewaschen, bis im Waschwasser kein Natrium oder Nitrat mehr nachweisbar war. Der so erhaltene Feststoff wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser verformt. Die Stränge wurden bei 100 bis 120°C getrocknet und anschließend 1 h bei 650°C und danach 3 h bei 850°C calziniert.

Der so hergestellte Katalysatorvorläufer enthielt 90,4 Gew.-% Kobalt, 5,1 Gew.-% Mangan, 0,3 Gew.-% Natrium und 3,1 Gew.-% Phosphor.

Anschließend wurde der Katalysator reduziert (H₂, 300 °C, 1 bar) und passiviert (N₂/O₂; 50 °C, 1 bar). In diesem Zustand wurden die Katalysatorformkörper in den Rohrreaktor eingebaut und die Laboranlage mit Stickstoff inertisiert.

Unter einem Wasserstoffstrom von 25 NI/h wurde binnen 12 h auf 280 °C geheizt, 12 h bei 280 °C gehalten und schließlich wurde der Reaktor unter einem Stickstoffstrom abgekühlt. Danach wurde der Druck auf 180 bar erhöht und es wurde innerhalb von 4 h eine Menge von 300 ml DMAPA im geraden Durchgang bei einem gleichzeitigen Wasserstoffstrom von 50 NI/h bei 50 °C von unten nach oben über den Katalysator gefahren.

Anschließend wurde die Hydrierung von DMAPN gestartet. Dazu wurden von unten nach oben 26.4 g/h DMAPN, 21.7 g/h flüssiger Ammoniak und 50 NI/h Wasserstoff über den Katalysator geführt. Die Starttemperatur bei allen Experimenten betrug 120 °C. Die Beheizung erfolgte mit Hilfe eines Öl-Thermostaten, die Temperaturmessung erfolgte im Ölbad des Mantels. Der Reaktor wurde in isothermer Weise betrieben.

Nach dem Durchgang durch den Reaktor wurde das Reaktionsgemisch bei 180 bar und Raumtemperatur in gasförmige und flüssige Komponenten aufgetrennt. Von den flüssigen Komponenten wurde mit Hilfe einer Kreislaufpumpe ein Strom von 80 g/h an den Eingang des Reaktors zurückgeführt. Gasförmige Komponenten wurden nicht rückgeführt. Der restliche Teil des Reaktionsgemisches wurde auf 20 bar entspannt und mit Hilfe eines Online-Gaschromatographen analysiert und schließlich bei 1 bar in einem Austragsgefäß aufgefangen.
GC-Säule: 60 m CP Volamine / WCOT Fused Silica 0,32 mm
Temperaturprogramm: 50°C - 10 min - 15°C/min - 240°C - 30 min
Die angegebenen DMAPN- Werte sind Gew.%.

Innerhalb der ersten sieben Tage, d.h. der ersten 168 h, wurde in den Experimenten die DMAPN-Zufuhr stufenweise auf 52.8 g/h und parallel die NH₃-Zufuhr auf 43.4 g/h erhöht. Nach diesem Zeitraum betrug die Katalysatorbelastung somit 2.0 kg DMAPN pro kg Katalysator pro Stunde.

Die anfängliche Temperatur von 120 °C wurde bei den Versuchen mit zunehmender Laufzeit erhöht, um den durch Katalysatordeaktivierung bedingten Umsatzrückgang zu kompensieren. Sobald die gaschromatographisch bestimmte DMAPN-Menge einen Wert von ca. 0.3 % überstieg, wurde die Temperatur im Ölmantel um 2 °C erhöht. Die Versuche wurden über eine Laufzeit von mind. 1500 h betrieben.

Die Katalysatordeaktivierung wird folgendermaßen quantifiziert:
Quotient aus: Erforderliche Temperaturerhöhung (über die Laufzeit, ausgehend von 120 °C Manteltemperatur, um einen Rest-DMAPN-Gehalt von ≤ 0.3 % zu gewährleisten) und Laufzeit. Dadurch wird angegeben, nach wie vielen Stunden im Mittel die Temperatur um 1 °C angehoben werden muss.

### Beispiel 1:

Es wurde das DMAPN aus Beispiel a) eingesetzt, welches einen Gehalt an 2-(Dimethylaminomethyl)-glutaronitril (DGN) von 227 ppm besaß.

Nach einer Versuchsdauer von 1600 h reichte eine Temperatur von 120 °C aus, um den Rest-DMAPN-Gehalt noch unter 0.3 % zu halten. Die Katalysatordesaktivierung lag somit unter 1 °C / 1000 h (120°C-120°C /1600 h < 1°C/1000 h).

### Beispiel 2 (Vergleichsbeispiel):

Es wurde das DMAPN aus Beispiel b) eingesetzt, welches einen Gehalt an 2-(Dimethylaminomethyl)-glutaronitril (DGN) von 373 ppm besaß.

Nach 1700 h musste eine Temperatur von 128 °C eingestellt werden, um den Rest-DMAPN-Gehalt bei ca. 0.3 % zu halten.

Die Katalysatordesaktivierung betrug somit 1°C/213 h (128°C-120°C/1700 h = 8°C/1700 h).

### Beispiel 3:

Es wurde das DMAPN aus Beispiel c) eingesetzt, welches einen Gehalt an 2-(Dimethylaminomethyl)-glutaronitril (DGN) von 27 ppm besaß.

Nach einer Versuchsdauer von 1600 h reichte eine Temperatur von 120 °C aus, um den Rest-DMAPN-Gehalt deutlich unter 0.3 % zu halten.

Die Katalysatordesaktivierung lag somit deutlich unter 1 °C /1000 h (120°C-120°C / 1700 h < 1 °C/1000 h).

Ein Vergleich der Beispiele 1 - 3 zeigt, dass die Desaktivierungsgeschwindigkeit des Katalysators in den Beispielen 1 und 3 am niedrigsten ist, während sie in Beispiel 2 am höchsten ist.

### Beispiel 4: Herstellung von DMAPN mit einem Gehalt an DGN von << 300 ppm durch Destillation

In einer Labor-Destillationsapparatur mit Vigreux-Kolonne wird DMAPN mit folgender Zusammensetzung (bzgl. organischer Komponenten) vorgelegt (GC-Analytik: Säule: 30 m RTX-5; ID = 0,32 mm, Filmdicke = 1,5 µm; Temperaturprogramm: 70 °C - 7 °C/min - 280 °C - 30 min; Auswertung in Flächenprozent):
96.97 % DMAPN
2.95 % Dimethylamin
0.02 % 2-(Dimethylaminomethyl)glutaronitril (200 ppm)
Der Wassergehalt betrug 0.3 %.
Nun wurde durch Destillation bei 100 mbar und Sumpf- sowie Kopftemperaturen von max.
100 °C ein geringer Anteil Destillat abgenommen, der zum überwiegenden Teil aus DMAPN bestand.

Die Zusammensetzung des verbliebenen Sumpfes war:
> 99.90 % DMAPN
< 0.01 % Dimethylamin
0.02 % 2-(Dimethylaminomethyl)glutaronitril
Der Wassergehalt betrug 0.02 %.

Nun wurde in einem zweiten Schritt das Sumpfprodukt bei 100 mbar und mindestens 100 °C Sumpf- und Kopftemperatur destilliert, bis nur noch eine geringe Menge Restsubstanz in der Destillationsblase zurückblieb. Vom Destillat wurde folgende Zusammensetzung bestimmt:
99.95 % DMAPN
2-(Dimethylaminomethyl)glutaronitril wurde nicht detektiert

Somit konnte ein DGN-freies DMAPN erhalten werden.

### Beispiel 5: Herstellung von DMAPN in Anlehnung an Beispiel 2 der WO-2007/128803 (Vergleichsbeispiel)

Die Anlagerung von DMA an ACN wurde in einer kontinuierlich betriebenen Rührkesselkaskade, bestehend aus sieben Rührkesseln, durchgeführt. Das Volumenverhältnis der sieben Rührkessel betrug 1:1 ,5:2, 5:3, 4:3, 4:3, 4:3, 4. Die Umsetzung wurde bei einem Druck von 5 bar durchgeführt.

Die Temperatur in den ersten zwei Rührkesseln betrug 60°C, im dritten bis siebten Kessel 40°C. Die Anlagerung wurde bei einer Belastung, gerechnet als Zulauf Acrylnitril pro Zeiteinheit, bezogen auf das Volumen des ersten Rührbehälters, von 0,54 kg/L/h durchgeführt. Das molare Einsatzstoffverhältnis von DMA zu ACN betrug 0,98. Der Acrylnitril-Restgehalt betrug ca. 2 Gew.-% (bezogen auf den gesamten Reaktionsaustrag). Die mittlere Verweilzeit des Reaktionsgemisches in den Behältern der Anlagerungsstufe betrug ca. 16 Stunden.

Eine Menge von 1406 g des so hergestellten DMAPN wurde wie oben beschrieben (siehe Beispiel a)) bis auf einen Rückstand von 10 g destilliert. Von diesem Rückstand wurde eine GC-Analyse durchgeführt (vgl. Beispiel a)):
DGN wurde bei einer Retentionszeit von 16 min detektiert.
Der Gehalt an DGN, bezogen auf die ursprünglich in die Destillation eingesetzte Menge DMAPN (1406 g) betrug 427 ppm.

## Patentansprüche

1. Verfahren zur Herstellung von 3-Dimethylaminopropylamin (DMAPA) durch Umsetzung von 3-Dimethylaminoprionitril (DMAPN) mit Wasserstoff in Gegenwart eines Katalysators, **dadurch gekennzeichnet, dass** das eingesetzte DMAPN einen Gehalt an 2-(Dimethylaminomethyl)-glutaronitril (DGN) von 300 Gew.-ppm oder weniger enthält, bezogen auf das eingesetzte DMAPN.

2. Verfahren nach Anspruch 1, **dadurch gekennzeichnet dass** das DMAPN einen Gehalt an DGN von 100 Gew.-ppm oder weniger enthält, bezogen auf das eingesetzte DMAPN.

3. Verfahren nach mindestens einem der Ansprüche 1 bis 2, **dadurch gekennzeichnet, dass** die Herstellung DMAPN mit einem DGN-Gehalt von 300 ppm oder weniger durch kontinuierliche Umsetzung von Acrylnitril mit Dimethylamin zu einem DMAPN-haltigen Reaktionsgemisch erfolgt und man das DMAPN-haltige Reaktionsgemisch destillativ aufarbeitet.

4. Verfahren nach Anspruch 3, **dadurch gekennzeichnet, dass** man die Destillation in einer Kolonnenverschaltung bestehend aus einer Kolonne für die Abtrennung von Leichtsiedern und einer weiteren Destillationskolonne zur Abtrennung der schwerer flüchtigen Nebenkomponenten durchführt.

5. Verfahren nach Anspruch 3, **dadurch gekennzeichnet, dass** man die Destillation in einer Kolonne durchführt, wobei Leichtsieder über Kopf, Schwersieder über Sumpf und DMAPN über einen gas- oder dampfförmigen Seitenabzug gewonnen werden.

6. Verfahren nach Anspruch 3, **dadurch gekennzeichnet, dass** man die Destillation in einer Kolonne mit Trennwand im mittleren Teil durchführt, bei der Leichtsieder über Kopf, Schwersieder über Sumpf und DMAPN auf der dem Zulauf gegenüberliegenden Seite der Trennwand gewonnen werden.

7. Verfahren nach Anspruch 1, **dadurch gekennzeichnet, dass** Dimethylaminoprionitril (DMAPN) durch Umsetzung von Dimethylamin und Acrylnitril in Gegenwart von Wasser in einer Reaktorkaskade in einem Temperaturbereich von 50 bis 80°C und einem Druckbereich von 3 bis 8 bar hergestellt wird, wobei das molare Verhältnis von DMA zu ACN im Bereich von 1,02:1 bis 1,10:1 liegt und der Anteil an Wasser im Bereich von 1 bis 8 Gew.-%, bezogen auf die Summe des eingesetzten DMAs und ACNs, liegt.

8. Verfahren nach mindestens einen der Ansprüche 1 bis 7, **dadurch gekennzeichnet, dass** der Katalysator als aktive Spezies Co oder Ni enthält.

9. Verfahren nach Anspruch 8, **dadurch gekennzeichnet, dass** der Katalysator durch Reduktion einer Sauerstoffverbindung von Co hergestellt wird.

10. Mischung von DMAPN und DGN, **dadurch gekennzeichnet, dass** das Gewichtsverhältnis von DMAPN zu DGN im Bereich von 1 000 000 : 5 bis 1 000 000 : 250 liegt.

11. Mischung gemäß Anspruch 10, **dadurch gekennzeichnet, dass** das Gewichtsverhältnis von DMAPN zu DGN im Bereich von 1 000 000 : 10 bis 1 000 000 : 100 liegt.

12. Verwendung einer Mischung gemäß Anspruch 10 oder 11 zur Herstellung von DMAPA.

## Claims

1. A process for preparing 3-dimethylaminopropylamine (DMAPA) by reacting 3-dimethylaminopropionitrile (DMAPN) with hydrogen in the presence of a catalyst, wherein the DMAPN used has a content of 2-(dimethylaminomethyl)glutaronitrile (DGN) of 300 ppm by weight or less, based on the DMAPN used.

2. The process according to claim 1, wherein the DMAPN has a DGN content of 100 ppm by weight or less, based on the DMAPN used.

3. The process according to at least one of claims 1 and 2, wherein the preparation of DMAPN having a DGN content of 300 ppm or less is carried out by continuously reacting acrylonitrile with dimethylamine to give a DMAPN-comprising reaction mixture and working up the DMAPN-comprising reaction mixture by distillation.

4. The process according to claim 3, wherein the distillation is carried out in a column arrangement comprising a column for separating off low boilers and a further distillation column for separating off the relatively nonvolatile secondary components.

5. The process according to claim 3, wherein the distillation is carried out in a column in which low boilers are obtained at the top, high boilers are obtained at the bottom and DMAPN is obtained via a gaseous side offtake stream.

6. The process according to claim 3, wherein the distillation is carried out in a column which has a dividing wall in the middle part and in which the low boilers are obtained at the top, high boilers are obtained at the bottom and DMAPN is obtained on the side of the dividing wall opposite the feed point.

7. The process according to claim 1, wherein dimethylaminopropionitrile (DMAPN) is prepared by reacting dimethylamine and acrylonitrile in the presence of water in a reactor cascade in a temperature range from 50 to 80°C and a pressure range from 3 to 8 bar, where the molar ratio of DMA to ACN is in the range from 1.02:1 to 1.10:1 and the proportion of water is in the range from 1 to 8% by weight, based on the sum of the DMA and ACN used.

8. The process according to at least one of claims 1 to 7, wherein the catalyst comprises Co or Ni as active species.

9. The process according to claim 8, wherein the catalyst is produced by reduction of an oxygen compound of Co.

10. A mixture of DMAPN and DGN, wherein the weight ratio of DMAPN to DGN is in the range from 1 000 000:5 to 1 000 000:250.

11. The mixture according to claim 10, wherein the weight ratio of DMAPN to DGN is in the range from 1 000 000:10 to 1 000 000:100.

12. The use of a mixture according to claim 10 or 11 for preparing DMAPA.

## Revendications

1. Procédé pour la préparation de 3-diméthylaminopropylamine (DRAPA) par transformation de 3-diméthylaminoprionitrile (DMAPN) avec de l'hydrogène en présence d'un catalyseur, **caractérisé en ce que** le DMAPN utilisé contient une teneur en 2-(diméthylaminométhyl)-glutaronitrile (DGN) de 300 ppm en poids ou moins, par rapport au DMAPN utilisé.

2. Procédé selon la revendication 1, **caractérisé en ce que** le DMAPN contient une teneur en DGN de 100 ppm en poids ou moins, par rapport au DMAPN utilisé.

3. Procédé selon au moins l'une quelconque des revendications 1 à 2, **caractérisé en ce que** la préparation de DMAPN présentant une teneur en DGN de 300 ppm ou moins a lieu par transformation continue d'acrylonitrile avec de la diméthylamine en un mélange réactionnel contenant du DMAPN et par traitement par distillation du mélange réactionnel contenant du DMAPN.

4. Procédé selon la revendication 3, **caractérisé en ce qu'**on réalise la distillation dans une commutation de colonnes, constituée par une colonne pour la séparation de fractions à bas point d'ébullition et une autre colonne de distillation pour la séparation de composants secondaires moins volatils.

5. Procédé selon la revendication 3, **caractérisé en ce qu'**on réalise la distillation dans une colonne, la fraction à bas point d'ébullition étant obtenue via la tête, la fraction à point d'ébullition élevé via le fond et le DMAPN via un soutirage latéral sous forme de gaz ou de vapeur.

6. Procédé selon la revendication 3, **caractérisé en ce qu'**on réalise la distillation dans une colonne présentant une paroi de séparation dans la partie centrale, la fraction à bas point d'ébullition étant obtenue via la tête, la fraction à point d'ébullition élevé via le fond et le DMAPN du côté opposé à l'alimentation de la paroi de séparation.

7. Procédé selon la revendication 1, **caractérisé en ce que** le diméthylaminoprionitrile (DMAPN) est préparé par transformation de diméthylamine et d'acrylonitrile en présence d'eau dans une cascade de réacteurs dans une plage de température de 50 à 80°C et une plage de pression de 3 à 8 bars, le rapport molaire de DMA à ACN se situant dans la plage de 1,02:1 à 1,10:1 et la proportion d'eau se situant dans la plage de 1 à 8% en poids, par rapport à la somme de DMA et d'ACN utilisés.

8. Procédé selon au moins l'une quelconque des revendications 1 à 7, **caractérisé en ce que** le catalyseur contient du Co ou du Ni comme substances actives.

9. Procédé selon la revendication 8, **caractérisé en ce que** le catalyseur est préparé par réduction d'un composé oxygéné de Co.

10. Mélange de DMAPN et de DGN, **caractérisé en ce que** le rapport pondéral du DMAPN à DGN se situe dans la plage de 1 000 000:5 à 1 000 000:250.

11. Mélange selon la revendication 10, **caractérisé en ce que** le rapport pondéral du DMAPN à DGN se situe dans la plage de 1 000 000:10 à 1 000 000:100.

12. Utilisation d'un mélange selon la revendication 10 ou 11 pour la préparation de DMAPA.
